# FASCICULE DE BREVET EUROPEEN

(11) **EP 3 727 320 B1**
(45) Date de publication et mention de la délivrance du brevet: **06.04.2022**
(21) Numéro de dépôt: 18842793.4
(22) Date de dépôt: 21.12.2018
(51) Int. Cl.: A61K 8/64, A61K 8/73, A61Q 19/00, A61K 36/00

(54) **COMPOSITION COSMÉTIQUE COMPRENANT UN EXTRAIT DE CAESALPINIA SPINOSA, UN EXTRAIT DE KAPPAPHYCUS ALVAREZII, ET UN HYDROLYSAT DE FÈVES DE THEOBROMA CACAO L**
KOSMETISCHE ZUSAMMENSETZUNG MIT EINEM EXTRAKT AUS CAESALPINIA SPINOSA, EINEM EXTRAKT AUS KAPPAPHYCUS ALVAREZII UND EINEM THEOBROMA-CACAO-L-BOHNENHYDROLYSAT
COSMETIC COMPOSITION COMPRISING AN EXTRACT OF CAESALPINIA SPINOSA, AN EXTRACT OF KAPPAPHYCUS ALVAREZII, AND A THEOBROMA CACAO L BEAN HYDROLYSATE

(30) Priorité: 22.12.2017 FR 1763141
(43) Date de publication de la demande: 28.10.2020
(73) Titulaire: L V M H RECHERCHE, 45800 St. Jean de Braye (FR)
(72) Inventeur: DUMAS, Marc, 45650 Saint Jean Le Blanc (FR); NOTTE, David, 45000 Orléans (FR); PELLICIER, Françoise, 45470 Loury (FR)
(74) Mandataire: Regimbeau
(86) Numéro de dépôt international: PCT/FR2018/053503
(87) Numéro de publication internationale: WO 2019/122777

(56) Documents cités:
- CN-A- 107 397 714

## Description

### DOMAINE DE L'INVENTION

La présente invention concerne une composition cosmétique comprenant un agent cosmétique constitué par des galactomannanes et des galactanes sulfatés réticulés, et un hydrolysat de fèves de *Theobroma cacao L,* et son utilisation notamment pour maintenir l'équilibre du microbiote cutané et favoriser et/ou améliorer la tenue du maquillage.

### ETAT DE LA TECHNIQUE

La peau, plus grand organe humain, est colonisée par des milliards de micro-organismes (bactéries, levures, champignons, virus...) collectivement appelés 'microbiote' ou 'microflore'. Le terme 'microbiome cutané' désigne quant à lui l'ensemble de ces micro-organismes, leur génome et leurs interactions avec leur environnement.

Le nombre de bactéries présentes sur la peau peut atteindre des millions par cm².

La flore cutanée humaine peut être subdivisée en deux groupes :
- La flore transitoire composée de champignons, virus et bactéries pour la plupart inoffensives, dites saprophytes, qui se nourrissent de matières organiques en décomposition provenant de l'environnement. Cette flore n'est pas permanente, elle varie dans la journée, selon les activités réalisées et les variations des conditions environnantes et l'exposition des individus à ces conditions. Les espèces transitoires les plus communes sont *Staphylococcus aureus, Escherichia coli, Pseudomonas aeruginosa* et des espèces *de Bacillus ;*
- La flore résidente est composée de germes commensaux, c'est-à-dire vivants grâce à leur hôte sans lui causer de dommage. La composition de cette flore est fixe, et après perturbations, ses mêmes composants se reforment spontanément. Ces micro-organismes (bactéries Gram positif, Gram négatif...) habitent l'épiderme et se retrouvent principalement dans les couches supérieures du *stratum corneum* ainsi que dans les conduits des glandes sudoripares et des follicules pilo-sébacés et les annexes épidermiques que sont les ongles et les cheveux.

Ces micro-organismes sont indispensables à la vie. Outre leur rôle dans l'apparition des odeurs corporelles, ils sont en effet étroitement liés au maintien d'un bon état de santé de la peau. Les microbes résidents et transitoires ne causent ni maladie ni dysfonctionnement dans des conditions normales, c'est-à-dire lorsqu'une hygiène adéquate est assurée et lorsque la flore résidente, les réponses immunitaires et la fonction barrière de la peau sont intactes. Le microbiote cutané est ainsi capable de jouer un rôle de barrière et de protéger son hôte.

En parallèle, l'épiderme génère des lipides et des peptides antimicrobiens, tels que les β-défensines, des récepteurs dédiés à la reconnaissance des pathogènes qui, tous ensemble forment 'l'immunité innée cutanée'.

En se développant et en se multipliant, les bactéries résidentes produisent par ailleurs des métabolites toxiques, les bactériocines, et des sérines protéases comme celles de *Staphylococcus epidermidis* permettant d'inhiber la croissance des autres micro-organismes et d'empêcher la formation de leur biofilm par lequel ils adhèrent à la peau..

La flore commensale stimule la barrière cutanée et l'immunité innée permettent ainsi le maintien d'une peau en bonne santé et résistante. L'équilibre du microbiote de la peau, ainsi que l'expression des conditions écologiques du milieu cutané (température, pH, salinité, teneurs hormonales, teneur en lipides et en protéines, en eau et en oxygène, exposition aux UV) sont également essentiels à ce maintien.

Outre les facteurs intrinsèques tels que l'âge, le sexe, la constitution génétique et la réactivité immunitaire, les facteurs extrinsèques tels que le climat (température, humidité ambiante, UV), les traitements médicamenteux, l'utilisation de produits cosmétiques d'hygiène, de soin ou de maquillage peuvent avoir un impact important sur la composition des communautés microbiennes cutanées.

Il a également été démontré que la composition du microbiote cutané est influencée par le la capacité de la peau à retenir l'eau qu'elle contient et la production de sébum. Le niveau de sébum est corrélé négativement avec les 3 indices de diversités à partir de données taxonomiques 16S (ordre, famille, genre et espèces) et la même tendance a été observée pour une perte insensible en eau (reflétant la qualité de la barrière cutanée).

Ces études présentées au Congrès de l'ASCB de San Francisco en décembre 2016 par C Heusèle et al. et intitulé 'Microbiota disversity on healthy women's face in relaton to skin biophysical characteristics', mettent en évidence une corrélation entre la production de sébum et la fonction barrière de la peau avec la composition et la diversité du microbiote.

La variabilité du microbiote cutané humain peut alors entraîner une altération de sa structure (dysbiose) et avoir des conséquences sur la santé en engendrant la survenue de dysfonctionnements cutanés ou contribuer au développement de pathologies via la colonisation et la prolifération de la flore résidente et/ou transitoire. Par exemple on observe une diminution de la diversité microbienne dans certaines pathologies comme la dermatite atopique (Flores G et al. J Drugs Dermatol 2014: 13(11): 1365-72) et le psoriasis (Alekseyenko A et al., Microbiome 2013 : 1(131) : 2-17)

L'application en particulier de compositions de maquillage, telles que des fonds de teint, peut ainsi perturber l'équilibre du microbiote cutané, en particulier en perturbant la diversité bactérienne. Le film invisible de maquillage appliqué sur la peau peut également générer transitoirement une modification de l'équilibre hydrolipidique de la peau et de la desquamation, facteurs susceptibles de déréguler l'équilibre du microbiote cutané.

Le film invisible de maquillage appliqué sur la peau peut aussi générer transitoirement une condition d'hypoxie susceptible de modifier le microbiote cutané, en particulier les microorganismes aérobies qui vivent à la surface du tégument où la teneur en oxygène est la plus élevée. Il a en effet été mis en évidence qu'une application de fond de teint impactait significativement la diversité bactérienne (Staudinger T et al. J Appl. Microbiol 2011 : 110 (6) : 1381-9.

Le film lipidique constitué par une production excessive de sébum peut également générer transitoirement une condition d'hypoxie susceptible de modifier le microbiote cutané, en particulier les microorganismes aérobies qui vivent à la surface du tégument où la teneur en oxygène est la plus élevée.

Mais l'hypoxie est également capable d'altérer le tissu cutané lui-même à plusieurs niveaux :
- en réduisant la formation de desmogleine-1 une protéine constitutive des liens de cohésion entre les cellules et donc de la résistance de l'épiderme (Straseski J et al. Wound Repair Regen. 2009; 17(4): 606-616),
- en diminuant aussi la formation de l'aquaporine 3, une protéine permettant l'apport d'eau dans l'épiderme pour hydrater la peau (Straseski J et al. Wound Repair Regen. 2009 ; 17(4): 606-616),
- en stimulant la production de molécules réactives de l'oxygène (ROS) qui altèrent les constituants et les cellules de la peau (Nys K et al. Free Radic Biol Med. 2012;52(6):1111-20)
- en inhibant la formation des kératines de différenciation comme la kératine 10 (Straseski J et al. Wound Repair Regen. 2009 ; 17(4): 606-616), et/ou
- en augmentant la présence des Matrix Metallo protéases ou MMP (Xia YP et al. J. Invest. Dermatol. 2001 ; 116 : 50-56) qui dégradent les constituants de la peau comme le collagène ce qui peut impacter la densité de la peau et sa fermeté et par conséquent l'ouverture des pores et la formation de rides et ridules.

On comprend donc l'intérêt, notamment pour des compositions de maquillage des matières kératiniques et notamment de la peau, ou des compositions pour peau grasse ou à tendance grasse de disposer d'ingrédients susceptibles de préserver l'écosystème cutané des conséquences de l'hypoxie en assurant une au moins des actions protectrices suivantes :
- en préservant le microbiote et sa diversité en lui apportant les conditions cutanées qui le permettent, telles que le renforcement de la barrière cutanée et le contrôle de la production de sébum,
- en renforçant la cohésion de l'épiderme pour renforcer la résistance de la peau,
- en stimulant la formation de la barrière cutanée pour maintenir une hydratation suffisante dans la peau,
- en détoxifiant la peau des espèces réactives de l'oxygène,
- en inhibant la formation des MMPs, et/ou
- en stimulant la formation de l'Hypoxia inducing factor ou HIF qui assure une protection des cellules de la peau de l'hypoxie.

Il subsiste donc le besoin de développer de nouvelles compostions cosmétiques de maquillage des matières kératiniques, en particulier pour la peau, capables notamment de préserver la diversité du microbiote, de contrôler son développement et d'améliorer la qualité des matières kératiniques (peau, muqueuses, phanères) et notamment la peau, le relief et le grain de sa surface qui constitue une niche écologique pour ces microorganismes, en particulier de maintenir l'écosystème cutané et/ou maintenir l'équilibre du microbiote cutané, en particulier la diversité microbienne, de renforcer la barrière cutanée, d'améliorer la résistance de la peau aux stress, en particulier prévenir et/ou limiter les conséquences cutanées d'un environnement hypoxique, de réduire l'inflammation cutanée, de favoriser la desquamation et/ou le renouvellement épidermique, de diminuer la perte de fermeté impliquée dans le relâchement des pores et la formation des rides et les ridules, d'améliorer l'homogénéité du teint, et/ou de favoriser et/ou améliorer la tenue du maquillage et l'homogénéité de sa répartition à la surface de la peau.

Le Demandeur a mis en évidence que l'association d'un agent cosmétique constitué par des galactomannanes et des galactanes sulfatés réticulés, et d'un hydrolysat de fèves de *Theobroma cacao L,* permettait de répondre à ce besoin. En particulier, le Demandeur a pu montrer qu'une composition comprenant un agent cosmétique constitué par des galactomannanes obtenus à partir de *Caesalpinia spinosa* de masses molaires comprises entre 5 et 630 kDa et de galactanes sulfatés réticulés obtenus à partir de *Kappaphycus alvarezii* de masses molaires comprises entre 7 et 3000 kDa et un hydrolysat de fèves de *Theobroma cacao L,* prévenait et/ou diminuer les effets du stress sur les kératinocytes, favorisait le renouvellement cellulaire, permettant ainsi d'améliorer la fonction barrière cutanée et maintenir l'équilibre du microbiote cutané.

### EXPOSE DE L'INVENTION

L'invention porte donc notamment sur une composition cosmétique comprenant, dans un milieu physiologiquement acceptable au moins :
a) un agent cosmétique constitué par des galactomannanes de masses molaires comprises entre 5 et 630kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un hydrolysat de fèves de *Theobroma cacao L.*

Un autre objet de l'invention vise un procédé cosmétique de soin et/ou de maquillage des matières kératiniques, en particulier de la peau, des lèvres et/ou des yeux, comprenant au moins l'application sur lesdites matières kératiniques, en particulier la peau du visage, d'une composition selon l'invention.

L'invention porte également sur l'utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa, et d'au moins un hydrolysat de fèves de Theobroma cacao L. tels que définis selon l'invention comme association destinée à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier prévenir et/ou limiter les conséquences cutanées d'un environnement hypoxique , favoriser la desquamation et/ou le renouvellement épidermique, diminuer la perte de fermeté, diminuer le relâchement des pores, diminuer les irrégularités de surface, améliorer l'homogénéité du teint, et/ou favoriser et/ou améliorer la tenue du maquillage et l'homogénéité de son dépôt.

Un autre objet de l'invention concerne au moins un agent cosmétique constitué par des galactomannanes de masses molaires comprises entre 5 et 630kDa et des galactanes sulfatés réticulés de masses molaires comprises entre 7 et 3000kDa, et d'au moins un hydrolysat de fèves de *Theobroma cacao L.* tels que définis selon l'invention ou d'une composition les contenant, pour une utilisation pour traiter et/ou diminuer les désordres liés à l'hyper-séborrhée, en particulier l'inflammation sébum-dépendante et/ou la prolifération bactérienne, en particulier chez les sujets à peaux grasses ou à tendance grasse.

### DEFINITIONS

Par matières kératiniques', on entend selon l'invention la peau, les muqueuses (en particulier les lèvres), et les phanères. Selon un mode particulier, la composition est destinée à une application topique sur la peau, les lèvres ou les phanères, de préférence la peau.

Par 'peau', on entend notamment la peau du visage et/ou du cou, en particulier la peau du visage, y compris les zones plus spécifiques des paupières (rentrant dans la catégorie de produits de maquillage des yeux) et du contour de l'œil.

Par microbiote cutané' ou 'microflore', on entend les micro-organismes (bactéries, levures, champignons, virus...) présents à la surface des matières kératiniques et notamment la peau.

Le terme 'microbiome cutané' désigne quant à lui l'ensemble de ces micro-organismes, leur génome et leurs interactions avec leur environnement.

Par écosystème cutané' autrement nommé biosphère cutanée', on entend selon l'invention le microbiote cutané et son hôte, la peau.

Par déséquilibre du microbiote cutané', on entend notamment un déséquilibre de la composition et/ou de la diversité du microbiote cutané.

Par maintenir l'écosystème cutané et/ou maintenir l'équilibre du microbiote cutané, en particulier la diversité microbienne', on entend selon l'invention la capacité des actifs ou d'une composition les contenant à préserver ou maintenir l'équilibre naturel de l'écosystème cutané, y compris en conditions de perturbations liées à des facteurs internes et/ou externes. Par maintenir l'équilibre du microbiote cutané', on entend notamment préserver ou maintenir la composition et/ou la diversité du microbiote, et contrôler son développement.

Par homogénéité du teint', on entend notamment un teint lumineux, homogène, on parle aussi d'éclat du teint, par opposition à altération du teint' qui désigne notamment un teint terne, inhomogène, brouillé, présentant des irrégularités de surface et/ou de couleur. Dans le cas des peaux grasses, l'altération du teint comprend en particulier un teint luisant et une perception d'inconfort et des manifestations ressenties comme des imperfections cutanées ou désordres esthétiques.

Par 'irrégularités de surface' autrement nommées 'imperfections cutanées', on entend notamment selon l'invention des irrégularités de relief (ex: les pores, les squames, les rides et ridules, un grain de peau imparfait) et/ou des irrégularités de couleur. Pour les lèvres, ces irrégularités de relief peuvent comprendre des gerçures ou crevasses.

Par prévenir et/ou limiter les conséquences cutanées d'un environnement hypoxique, on entend notamment selon l'invention maintenir la diversité du microbiote, et/ou prévenir et/ou améliorer les altérations de la peau liées à une condition transitoire d'hypoxie, telles qu'une diminution de la cohésion cellulaire, une diminution de l'apport en eau dans l'épiderme, une augmentation de la production des molécules réactives de l'oxygène, une inhibition de la formation des kératines de différenciation et/ou une augmentation de la dégradation du collagène.

Par 'peau grasse ou à tendance grasse', on entend notamment une peau grasse caractérisée par une présence excessive en sa surface de sébum, produit de sécrétion des glandes sébacées. Cette production de sébum est assurée plus particulièrement par les sébocytes au travers d'un processus de différenciation cellulaire et de synthèse ou accumulation de lipides appelé lipogenèse. On parle également de peau hyperséborrhéique.

Une peau grasse est souvent associée à un défaut de desquamation, un terrain inflammatoire et/ou une oxydation des lipides, pouvant provoquer un teint luisant, un grain de peau imparfait, une perception d'inconfort et manifestations ressenties comme des imperfections cutanées ou désordres esthétiques ; une telle peau présente également une moins bonne tenue du maquillage au cours de la journée et particulièrement l'après-midi lorsque la production sébacée est maximale.

Outre son aspect disgracieux, une peau grasse peut être un terrain favorable à la prolifération de micro-organismes et participer ainsi à la survenue de désordres cutanés (ex : lésions d'acné). On parlera alors de peau grasse ou hyperséborrhéique à tendance acnéique.

Par désordres esthétiques cutanés ou désordres non pathologiques associés aux peaux grasses ou à tendance grasse', on entend notamment des désordres dus à une hyper-séborrhée, un défaut de desquamation, un environnement hypoxique et/ou à un déséquilibre du microbiote. En particulier, les désordres esthétiques cutanés associés sont des désordres non pathologiques sélectionnés parmi une peau présentant un déséquilibre du microbiote cutané, un défaut de desquamation, une oxydation des lipides, une altération du teint en particulier un teint luisant, une perception d'inconfort, des irrégularités de surface, en particulier des orifices folliculaires ou des pores dilatés, un grain de peau imparfait, et/ou une peau présentant une moins bonne tenue du maquillage.

### DESCRIPTION DETAILLEE DE L'INVENTION

Un premier objet de l'invention est donc une composition cosmétique comprenant, dans un milieu physiologiquement acceptable au moins :
a) un agent cosmétique constitué par des galactomannanes de masses molaires comprises entre 5 et 630kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un hydrolysat de fèves de *Theobroma cacao L.*

### Galactomannanes et Galactanes sulfatés réticulés

L'agent cosmétique au sens de l'invention est constitué de biopolymères, c'est-à-dire de polymères issus de matières végétales, qui sont obtenus par synthèse chimique.

Ces biopolymères ont un effet filmogène, c'est-dire un effet pouvant créer à la surface de la peau un film non perceptible à l'œil nu, afin de protéger la peau des agressions externes telle que la pollution et les allergènes.

De tels biopolymères sont décrits notamment dans les demandes de brevets WO2017/1 2 9792 et WO2017/129780 de la Société Industrielle Limousine d'Application Biologique.

L'agent cosmétique ou dermocosmétique utilisé selon l'invention est constitué par :
- des galactomannanes de masses molaires comprises entre 5 et 630kDa, et
- des galactanes sulfatés réticulés de masses molaires comprises entre 7 et 3000kDa.

Par 'masse molaire moyenne' d'un mélange de molécules, on entend la moyenne des masses molaires pondérales de chaque molécule du mélange.

Par 'réticulé', on entend au sens de l'invention un biopolymère dans lequel a été formé un réseau tridimensionnel au moyen de la formation de liaisons chimiques ou physiques entre les molécules du biopolymère.

Préférentiellement, l'agent cosmétique ou dermocosmétique est constitué par :
- des galactomannanes de masse molaire moyenne comprise entre 5 et 120kDa, et des galactanes sulfatés réticulés de masse molaire moyenne comprise entre 7 et 1100kDa.

Encore plus préférentiellement l'agent cosmétique ou dermocosmétique est constitué par :
- des galactomannanes de masse molaire moyenne comprise entre 8 et 80kDa, et des galactanes sulfatés réticulés de masse molaire moyenne comprise entre 8 et 200kDa.

Les galactomannanes peuvent être obtenus notamment à partir d'hydrolyse de galactomannanes natifs de Tara (*Caesalpinia spinosa*)*,* de guar (*Cyamopsis tetragonoloba*), de caroube (*Ceratonia siliqua*)*,* mais aussi de séné (Cassia *angustifolia*)*,* de cassier (Cassia *fistula*), de cassia (Cassia *obtusifolia* ou Cassia *tora*), de caroube chinois (*Gleditsia sinensis*), de févier (*Gieditsia triacanthos*), de sophora (*Sophora japonica*) et/ou de fenugrec (*Trigonella foenum-graecum*), de préférence de *Caesalpinia spinosa.*

Les galactanes sulfatés réticulés peuvent être obtenus par hydrolyse de galactanes sulfatés natifs de carraghénanes (de *Kappaphycus alvarezii,* de *Kappaphycus striatum, d'Eucheuma cottonii, d'Eucheuma spinosum,* de *Chondrus crispus,* de *Gigartina skottsbergii,* de *Sacrothalia crsipata*)*,* ou de *Fucellaria fastigiata,* d'Agar (*Gelidium sesquipedale*) ou des algues (*Polysiphonia lanosa* ou *Codium fragile*), de préférence de *Kappaphycus alvarezii.* Selon un mode particulier de l'invention, l'agent cosmétique est constitué de galactomannanes obtenus par hydrolyse de galactomannanes natifs de Tara (*Caesalpinia* spinosa), de guar (Cyamopsis tetragonoloba), de caroube (Ceratonia siliqua), mais aussi de séné (Cassia angustifolia), de cassier (Cassia fistula), de cassia (Cassia *obtusifolia* ou Cassia tora), de caroube chinois (Gleditsia sinensis), de févier (Gieditsia triacanthos), de sophora (Sophora japonica) et/ou de fenugrec *(Trigonella* foenum-graecum), de préférence de *Caesalpinia* spinosa ; et de galactanes sulfatés réticulés obtenus par hydrolyse de galactanes sulfatés natifs de carraghénanes (de Kappaphycus alvarezii, de Kappaphycus striatum, d'Eucheuma cottonii, d'Eucheuma spinosum, de Chondrus crispus, de Gigartina skottsbergii, de *Sacrothalia* crsipata), ou de Fucellaria fastigiata, d'Agar (Gelidium sesquipedale) ou des algues (*Polysiphonia* lanosa ou Codium fragile), de préférence de Kappaphycus alvarezii.

Les galactomannanes et galactanes sulfatés réticulés sont obtenus selon les étapes suivantes :
- Solubilisation de poudre de galactomannanes natifs ou respectivement des galactanes sulfatés natifs dans l'eau,
- Hydrolyse ménagée pat voie chimique ou enzymatique ;
- Séparation des phases solubles ou insolubles, afin d'éliminer la phase insoluble ;
- Sélection par filtration(s) membranaire(s) des galactomannanes de masse molaire définie.

Les galactanes sulfatés réticulés sont aussi réticulés par un agent réticulant, préférentiellement un agent réticulant de nature ionique.

Les galactomannanes et galactanes sulfatés réticulés ainsi obtenus sont mélangés pour constituer un agent cosmétique.

Selon un mode particulier et préféré, l'agent cosmétique a) selon l'invention est constitué de 60 à 90% de galactomannanes et 10 à 40% de galactanes sulfatés réticulés.

Selon un mode particulier et préféré, l'agent cosmétique est constitué de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises notamment entre 1 et 150kDa.

Selon un mode particulier de l'invention, l'agent cosmétique a) est présent dans la composition en une teneur allant de 0,01% à 2% en poids de matière active par rapport au poids total de la composition, préférentiellement 0,1% à 0,1%, et préférentiellement encore de 0,4% à 0,8% en poids de matière active par rapport au poids total de la composition.

Par matière active', on entend les composés actifs de l'agent cosmétique auxquels est attribuée l'efficacité. On parle également de matière sèche pour les extraits végétaux.

De tels biopolymères sont décrits dans les demandes de brevets WO2017/19792 et WO2017/129780 de la Société Industrielle Limousine d'Application Biologique, en particulier l'exemple 1 de la demande WO2017/129780. L'utilisation de ces biopolymères permet d'améliorer l'effet barrière cutané par un effet protecteur de la peau contre la pénétration de molécules toxiques, telles que les polluants, les allergènes, les métaux lourds. L'effet de film après application su la peau permet également d'améliorer l'apparence globale du visage et favoriser la tenue des pigments et du maquillage.

Selon un mode particulier et préféré, on utilisera comme agent cosmétique constitué par des galactomannanes et des galactanes sulftates réticulé un produit commercialisé par la société SILAB sous la dénomination commerciale FILMEXEL^{®}, de nom INCI 'CAESALPINIA SPINOSA EXTRACT and KAPPAPHYCUS ALVAREZII EXTRACT and WATER', sous forme de poudre et de composition CAESALPINIA SPINOSA EXTRACT 76%, KAPPAPHYCUS ALVAREZII EXTRACT 19%, and WATER 5% (95% de matière active ou matière sèche).

### Hydrolysat de Theobroma cacao L

L'hydrolysat de *Theobroma cacao L.* selon l'invention est un hydrolysat peptidique et osidique des fèves de *Theobroma cacao L* comprenant majoritairement des peptides et des osides. On entend par "majoritairement des peptides et des osides" une quantité supérieure à 50%, préférentiellement supérieure à 60%, préférentiellement encore supérieure à 70% et pouvant atteindre environ 90% (poids/poids) de matière sèche en peptides et en oses, préférentiellement 90% du poids de la matière sèche.

On entend par "hydrolysat peptidique et osidique" un hydrolysat comprenant majoritairement ou essentiellement des peptides et des oses (mono et oligosaccharides). Les protéines et les polysaccharides naturellement présents dans les fèves ont été hydrolysés en peptides, en oligosaccharides et en monosaccharides, avantageusement l'hydrolyse est une hydrolyse enzymatique.

Cet hydrolysat peut être obtenu comme suit :
a) on met en dispersion en phase aqueuse des fèves broyées de *Theobroma cacao L ;*
b) on effectue un traitement enzymatique de la dispersion aqueuse obtenue à l'étape a) ;
c) on effectue la récupération de l'hydrolysat enzymatique par séparation solide / liquide,
d) on purifie l'hydrolysat par ultrafiltration et nanofiltration, puis éventuellement ; e) on effectue une lyophilisation de l'hydrolysat obtenu à l'étape d).

L'hydrolysat utilisé selon l'invention est obtenu à partir de fèves de *Theobroma cacao L,* comme matière de départ, qui peuvent comprendre indifféremment la fève seule ou la fève et son enveloppe, préférentiellement on utilisera les fèves comprenant la fève et son enveloppe. L'hydrolysat de cacao non lyophilisé utilisé selon l'invention comprend de 20 à 70% de peptides et de 5 à 40% d'osides. L'hydrolysat de cacao lyophilisé sans support de séchage selon l'invention comprend plus de 90 pourcent de matière sèche comprenant de 20 à 70% de peptides et de 5 à 40% d'osides.

Dans un autre mode de réalisation très préféré selon l'invention, les peptides et osides présents dans l'hydrolysat utilisé selon l'invention présentent un poids moléculaire compris entre 200 Da et 10 kDa.

Ainsi, selon un mode particulier et préféré, l'hydrolysat de fèves de Theobroma cacao L est un hydrolysat peptidique et osidique de fèves de Theobroma cacao L, comprenant notamment peptides et osides présentant un poids moléculaire compris entre 200 Da et 10 kDa.

L'hydrolysat ainsi obtenu peut ensuite être encore dilué dans de l'eau ou dans tout mélange de solvants contenant de l'eau. Ainsi, l'hydrolysat de cacao selon l'invention peut avantageusement être dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. Pour préparer la composition, l'hydrolysat de cacao selon l'invention peut se trouver sous forme liquide ou lyophylisée. Selon un mode particulier, il est sous une forme liquide.

Selon un mode particulier et préféré, on utilisera comme hydrolysat de Theobroma cacao L. un produit commercialisé par la société ASHLAN sous la dénomination commerciale BLUMILIGHT^{™}, de nom INCI 'BUTYLENE GLYCOL and WATER and THEOBROMA CACAO (COCOA) SEED EXTRACT', sous forme d'un liquide et de composition BUTYLENE GLYCOL 55%, WATER 43.75%,THEOBROMA CACAO (COCOA) SEED EXTRACT 1.25% (1.25% de matière active ou matière sèche).

Selon un mode particulier, l'hydrolysat de fèves de Theobroma cacao L. est présent dans la composition en une teneur allant de 0,0005 à 0,025% en poids de matière active par rapport au poids total de la composition, préférentiellement de 0,001 à 0,01% en poids de matière active par rapport au poids total de la composition.

Selon un mode particulier de l'invention, la composition cosmétique comprend en outre un prébiotique et un probiotique. On parle aussi de synbiotique ou complexe prébiotique/probiotique pour définir l'association d'un prébiotique et d'un probiotique.

### Complexe prébiotiaue/probiotiaue (synbiotique)

On entend par « prébiotique » des constituants alimentaires non vivants qui ont la propriété de moduler la croissance et l'activité des souches probiotiques (lactobacilles, bifidobactéries...) et des autres souches bactériennes de l'intestin bénéfiques à la santé humaine. Les prébiotiques sont généralement des monosaccharides, disaccharides ou oligosaccharides, qui peuvent se retrouver naturellement dans les fruits, les légumes, et le miel et sont extractibles. D'autres sont produits industriellement par hydrolyse des polysaccharides (par exemple des fructo-oligosides ou oligofructoses, abrégé en FOS pour Fructo-oligosaccharides) ou synthétisés en soumettant des disaccharides tels que le lactose à l'action d'enzymes comme les lactases avec des activités transférases pour produire des trans-galacto-oligosaccharides (TOS) ou par une réaction chimique d'isomérisation qui donne lelactulose. Actuellement, les trans-galactooligosaccharides (GOS) et les fructanes de type inuline sont ceux dont les effets prébiotiques sont reconnus.

On entend par « probiotique ou dérivé » des micro-organismes vivants ou inactivés qui, lorsqu'ils sont ingérés en quantité suffisante, exercent des effets positifs sur la santé au-delà des effets nutritionnels traditionnels.

Les probiotiques peuvent être des bactéries ou des levures.

Le probiotique ou dérivé peut être choisi parmi une souche d'un ou plusieurs des suivants: *Lactobacillus,* souches et dérivés de *Clostridia,* souches et dérivés de *Bifidobacterium,* souches et dérivés de *Saccharomyces,* souches et dérivés de *Lactococcus,* souches et dérivés de *Pedicoccus,* souches et dérivés de Enterococcus, souches et dérivés *d'Escherichia,* souches et dérivés *d'Alcaligenes,* souches et dérivés de *Corynebacterium,* souches et dérivés de *Bacillus,* et souches et dérivés de *Propionibacterium.*

On peut citer notamment les bactéries des genres *Lactobacillus* et *Bifidobacterium.* Les souches identifiées comme probiotiques s'appliquent principalement à la sphère intestinale, il s'agit notamment de *Lactobacillus acidophilus, L. casei, L. gasseri, L. paracasei, L. Rhamnosus* et *Bifidobacteria animal, B. breve, B. longum.*

Comme levures, on peut citer notamment *Saccharomyces cerevisiae var. boulardii* qui se trouve de façon naturelle dans le litchi.

On entend par 'synbiotiques' des associations de probiotiques et de prébiotiques.

Selon un mode particulier, le prébiotique est choisi dans le groupe constitué par l'inuline et l'alphaglucane oligosaccharide.

Selon un mode particulier, le probiotique est choisie dans le groupe constitué par des lysats de probiotiques. De préférence, le lysat de probiotique est un lysat du genre *Bacillus.*

Selon un mode particulier et préféré, la composition cosmétique de l'invention comprend comme complexe prébiotique/probiotique Ecoskin^{®} commercialisé par la société SOLABIA, qui est un mélange d'oligosaccharide d'alpha-glucane, de jus de racine de sonchifolia de polymnia, de maltodextrine, et de bactéries de *Lactobacillus sp,* de nom INCI :
ALPHA-GLUCAN OLIGOSACCHARIDE and POLYMNIA SONCHIFOLIA ROOT JUICE and MALTODEXTRIN and LACTOBACILLUS
La composition de Ecoskin^{®} est : ALPHA-GLUCAN OLIGOSACCHARIDE 70%, POLYMNIA SONCHIFOLIA ROOT JUICE 19%, MALTODEXTRIN 10%, LACTOBACILLUS 1% (correspondant à 90% de matière active).

C'est un complexe pré/probiotique, séché par pulvérisation sur la maltodextrine, fait de: α-gluco-oligosaccharides (GOS) obtenu par synthèse enzymatique à partir de substrats végétaux (maltose de maïs, saccharose de betterave), 100% de jus végétaux purs riches en β-fructooligosaccharides (FOS) obtenus par pressage à froid de tubercules de *Polymnia sonchifolia,* et d'une bactérie probiotique de *Lactobacillus (L. casei, L. acidophilus),* inactivée par tyndallisation et lyophilisée.

La portion prébiotique provient des α-gluco-oligosaccharides (GOS) et des β-fructooligosaccharides de l'extrait pressé à froid de tubercules de *Polymnia sonchifolia* ; ces prébiotiques stimulent l'écoflore de la peau.

Les probiotiques sont les bactéries *Lactobacillus* inactivées qui stimulent les β-défensines qui sont impliquées dans le système de défense de la peau. Les souches de bactéries *Lactobacillus* (*Lactobacillus casei* et *Lactobacillus acidophilus*) qui sont utilisées dans Ecoskin ^{®} sont auparavant lyophilisées et tyndallisées, ce qui signifie que leur système de reproduction est inactivé par la chaleur empêchant leur développement dans les préparations cosmétiques qui les contiennent.

Selon un mode particulier, le complexe prébiotique/probiotique est présent dans la composition en une teneur allant de 0,05% à 3% en poids de matière active par rapport au poids total de la composition, préférentiellement de 0,1 à 1% en poids de matière active par rapport au poids total de la composition.

### Galénique

Les compositions selon l'invention sont destinées plus particulièrement à une application topique sur les matières kératiniques, en particulier sur la peau.

Les compositions de l'invention comprennent un milieu cosmétiquement acceptable, c'est-à-dire compatible avec la peau et les phanères. Les compositions peuvent avoir toutes les formes cosmétiques, et notamment être sous forme de crèmes, émulsions huile-dans-eau, ou eau-dans-huile ou émulsions multiples, solutions, suspensions, gels, laits, lotions, sérums, sticks ou encore de poudres, et adaptées à une application sur la peau, les lèvres et/ou les phanères.

Selon un mode particulier, la composition de l'invention est destinée à une application topique sur la peau, les lèvres ou les yeux et se présente sous la forme d'un produit de soin et/ou d'un produit de maquillage pour la peau, les lèvres et ou les yeux, en particulier pour le teint.

On parlera indifféremment de maquillage de la peau ou maquillage pour le teint.

Par 'maquillage des yeux', on entend notamment des liners, ou des fards à paupières.

Selon un mode particulier et préféré, la composition de l'invention se présente sous la forme d'un fond de teint, ou d'une base de teint, de préférence d'un fond de teint.

Selon un mode particulier et préféré, la composition de l'invention se présente sous la forme d'une poudre de teint, d'un fond de teint, ou d'une base de teint, de préférence d'un fond de teint.

La composition cosmétique de l'invention est avantageusement sous la forme d'une émulsion huile-dans-eau, une émulsion eau-dans-huile, une émulsion multiple, un gel aqueux.

La composition est de préférence sous la forme d'une émulsion contenant une phase aqueuse et une phase huileuse.

La phase aqueuse représente généralement de 1 à 99% en poids, par rapport au poids total de ladite composition.

La composition de l'invention comprend généralement, outre la phase aqueuse, également une phase huileuse.

Une phase huileuse selon l'invention peut comprendre des huiles hydrocarbonées, siliconées, fluorées ou non, et leurs mélanges. Ces huiles peuvent être volatiles ou non volatiles, végétale, minérale ou synthétique.

Avantageusement, on utilisera des huiles hydrocarbonées.

Comme huiles volatiles hydrocarbonées, on peut citer notamment les alcanes ramifiés en C8-C16, les esters ramifiés en C8-C16 et leurs mélanges.

Comme huiles non volatiles hydrocarbonées, on peut citer notamment les huiles hydrocarbonées, les huiles hydrocarbonées d'origine végétale, les éthers de synthèse en C10-C40, les esters de synthèse en C10-C40, les alcools gras en C12-C26, les acides gras supérieurs en C12-C22, et leurs mélanges.

Les huiles pourront être présentes dans la composition de l'invention en une teneur allant de 1 à 95 % en poids par rapport au poids total de la composition.

La composition de l'invention peut également comprendre tout additif usuellement utilisé en cosmétique tels que des filtres UV, des antioxydants, des tensio-actifs, des gélifiants, des charges, matières colorantes, des conservateurs, des polymères filmogènes, des parfums, des agents actifs cosmétiques, comme par exemple des émollients, des hydratants, des vitamines, des agents anti-âge, des agents éclaircissants, et leurs mélanges.

Selon un mode particulier de l'invention, la composition cosmétique comprend au moins une ou plusieurs matières colorantes.

Les matières colorantes peuvent être choisies parmi les matières colorantes hydrosolubles ou non, liposolubles ou non, organiques ou inorganiques, les matériaux à effet optique, et leurs mélanges.

On entend par matière colorante au sens de la présente invention, un composé susceptible de produire un effet optique coloré lorsqu'il est formulé en quantité suffisante dans un milieu cosmétique approprié.

Selon un mode particulier, la ou les matières colorantes sont notamment choisies parmi des pigments minéraux et/ou organiques, des pigments composites (à base de matériaux minéraux et/ou organiques), des colorants, des nacres ou pigments nacrés, et leurs mélanges.

Par pigments, il faut comprendre des particules blanches ou colorées, inorganiques (minérales) ou organiques, insolubles dans la phase organique liquide, destinées à colorer et/ou opacifier la composition et/ou le dépôt réalisé avec la composition.

Parmi les pigments minéraux, on peut citer, à titre d'exemples le dioxyde de titane (rutile ou anatase), éventuellement traité en surface ; les oxydes de fer noir, jaune, rouge et brun ; le violet de manganèse ; le bleu outremer l'oxyde de chrome l'oxyde de chrome hydraté et le bleu ferrique.

Pour les compositions destinées aux lèvres, on peut citer à titre d'exemples le dioxyde de titane; les oxydes de fer noir, jaune, rouge et brun et le violet de manganèse.

Parmi les pigments organiques, on peut citer, par exemple, les pigments D & C red n° 19; D & C red n° 9; D & C Red n° 22 ; D & C Red n° 21 ; D & C Red n° 28 ; D & C Yellow n° 6 ; D & C orange n° 4 ; D & C orange n° 5 ; D & C Red n° 27; D & C red n° 13; D & C Red n° 7 ; D & C Red n° 6 ; D & C Yellow n° 5; D & C Red n° 36 ;; D & C Red n° 33 ; D & C orange n° 10; D & C yellow n° 6 ; ; D & C Red n° 30 ; D &C red n° 3 ; D &C Blue 1 ; le noir de carbone et les laques à base de carmin de cochenille.

Parmi les colorants hydrosolubles, on pourra citer Yellow 5, Yellow 6, Blue 1, Green 5, Green 3, Green 6, Orange 4, Red 4, Red 21,Red 22, Red 27, Red 28, Red 33, Red 40, le carminé de cochenille (Cl 15850, Cl 75470).

Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

Les nacres ou pigments nacrés peuvent être choisis notamment parmi les pigments nacrés blancs, tels que le mica recouvert d'oxyde de titane, l'oxychlorure de bismuth ; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth. On pourra citer les gammes de nacres de référence commerciale Reflecks^{®}, Ronastar^{®}, Timiron^{®} et Syncristal^{®}.

En particulier, la ou les matières colorantes sont présentes dans la composition en une teneur allant de 2% à 30% en poids, de préférence de 4% à 15% en poids par rapport au poids total de la composition.

Selon un mode particulier, la composition de l'invention comprend des pigments, en particulier des pigments minéraux en une teneur allant de 5 à 25%, notamment de 10 à 20% en poids par rapport au poids total de la composition.

Les charges sont choisies notamment parmi les silices, les micas, d'origine naturelle ou synthétique, le kaolin, les oxydes de zinc et de titane ; le carbonate de calcium, le carbonate et l'hydrocarbonate de magnésium ; le stéarate de zinc, de magnésium ou de lithium, le laurate de zinc, le myristate de magnésium ; les poudres de polymères synthétiques, tels que le polyéthylène, les polyesters, les polyamides (par exemple le nylon) ; des poudres d'acides polyacrylique ou polyméthacrylique, des poudres de résine de silicone ; les poudres minérales telles que la silice sphérique ; les dioxydes de titane sphériques; les billes de verre et de céramique ; des poudres de matériaux organiques d'origine naturelle comme les amidons de maïs, de blé, de riz, réticulées ou non, et leurs mélanges.

Selon un mode particulier, la composition de l'invention comprend des pigments, en particulier des pigments minéraux en une teneur allant de 5 à 25%, notamment de 10 à 20% en poids par rapport au poids total de la composition.

Selon un mode particulier de l'invention, la composition cosmétique ne contient pas d'autres actifs cosmétiques que ceux objets de l'invention.

Selon un autre mode particulier de l'invention, la composition contient en outre d'autres actifs cosmétiques et/ou dermatologiques. On peut citer notamment les actifs visant à stimuler la biodiversité et/ou l'homéostasie du microbiote cutané, le renouvellement cellulaire, la régénération ou revitalisation du teint (éclat), la réduction de la production de sébum, la protection vis-à-vis d'agressions externes.

On peut citer notamment les actifs suivants visant à stimuler :
- la biodiversité et l'homéostasie du microbiote de la peau, comme le fait l'actif Actibiome^{®} (Water and seawater and glycerin and Laminaria digitata extract and Chlorella vulgaris extract and saccharide isomerate and phenoxyethanol and ethylhexylglycerin), en rééquilibrant le pH de la peau précédemment diminué par une période de stress ;
- le renouvellement cellulaire, avec l'aide d'actifs à effet « peeling » seuls ou en combinaison et à pH acide :
   - par voie chimique, comme les Acides Alpha Hydroxylés AHA (acide glycolique, lactique, citrique...), Acides Beta Hydroxylés BHA (acide salicylique), Acides Poly Hydroxylés PHA (gluconolactone) ;
   - par voie biologique, comme Exfolactive C EL PX^{©} (Opuntia coccinellifera), DERMOCH DP^{©} (Chlorella vulgaris), ALGOWHITE^{©} (Ascophyllum nodosum)
   - par voie enzymatique, comme MELACLEAR^{©} (Gluconic acid, Sutilains) ;
- la régénération ou revitalisation du teint (éclat), à l'aide d'actifs :
   - lissants comme le Retinol (vitamine A-like)
   - éclaircissants par action sur la mélanogénèse (dérivé de vitamine C)
   - énergisants tels que des nutriments et/ou anti-oxydants vitaminiques C et E comme SEPIVITAL^{©} (potassium ascorbyl tocopheryl phosphate)
- la réduction de la production de sébum, à l'aide d'agents sébo-capteurs (Argiles) ou actifs sébo-régulateurs comme le gluconate de Zinc (Mineralis GU / Zn) et l'extrait lipophyle d'avocat (5 alpha avocuta),
- la protection vis-à-vis d'agressions externes : pollution, lumière bleue, UV à l'aide de filtres chimiques ou minéraux et/ou particules réfléchissantes diffusantes,
et leurs mélanges.

L'invention porte également sur un procédé cosmétique de soin et/ou de maquillage des matières kératiniques, en particulier de la peau, des lèvres et/ou des yeux, comprenant au moins l'application sur lesdites matières kératiniques, en particulier la peau du visage, d'une composition selon l'invention.

Selon un mode particulier, il s'agit d'une composition de maquillage pour la peau, les lèvres ou les yeux, de préférence pour le teint.

Selon un mode particulier, il s'agit d'un procédé de soin et/ou de maquillage de la peau, en particulier de la peau du visage et/ou du cou.

Selon un mode particulier, il s'agit d'un procédé de soin et/ou de maquillage des lèvres. Selon un autre mode particulier, il s'agit d'un procédé de de soin et/ou de maquillage des yeux, en particulier des paupières.

Selon un mode particulier, le procédé est destiné à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier prévenir et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, et/ou favoriser et/ou améliorer la tenue du maquillage et l'homogénéité de son dépôt.

Un autre objet de l'invention est l'utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa, et d'au moins un hydrolysat de fèves de *Theobroma cacao L.* tel que défini précédemment, comme association destinée maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier prévenir et/ou limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, favoriser et/ou améliorer la tenue du maquillage et l'homogénéité de son dépôt.

L'invention porte encore sur au moins un agent cosmétique constitué par des galactomannanes de masses molaires comprises entre 5 et 630kDa et des galactanes sulfatés réticulés de masses molaires comprises entre 7 et 3000kDa, et d'au moins un hydrolysat de fèves de *Theobroma cacao L.* tel que défini précédemment ou d'une composition les contenant, pour une utilisation pour traiter et/ou diminuer les désordres liés à l'hyper-séborrhée, en particulier l'inflammation sébum-dépendante et/ou la prolifération bactérienne, en particulier chez les sujets à peaux grasses ou à tendance grasse.

L'invention va être illustrée par les exemples non limitatifs suivants. Les pourcentages sont exprimés en pourcentage en poids par rapport au poids total de la composition sauf indication contraire.

### EXEMPLES

### Exemple 1 : Effet de l'association d'un extrait de Caesaloinia spinosa et d'un extrait de Kappaphvcus alvarezii (Filmexel^{®} ) en combinaison avec hydrolysat de fèves de Theobroma cacao L (Blumilight^{™}) sur les kératinocvtes

Une étude sur les effets transcriptionnels de ces actifs, utilisés seuls ou en association, a été réalisée à l'aide de la technologie TLDA (TaqMan Low Density Array). Grâce à cette technologie, la modulation de l'expression de gènes codant pour des protéines spécifiques est étudiée en réponse à un traitement de Kératinocytes Humains Normaux, ce qui permet une cartographie de l'expression génique des actifs seuls ou en association.

Cette approche permet, notamment, de mettre en évidence les effets bénéfiques des actifs ou de leur combinaison dans les domaines couverts par le profil : formation de l'épiderme et desquamation, matrice extracellulaire, détoxification et anti-oxydation, inflammation et immunité innée.

### Matériel et méthodes

Cellules utilisées : Kératinocytes normaux d'épiderme humain
Milieu de culture : EpiLife^{®} complété par du HKGS (Human-Keratinocyte-Growth-Supplement)
Origine cutanée des cellules : prélèvement abdominal, femme, 47 ans
Modèle d'étude : Technologie TLDA (TaqMan Low Density Array)
Traitement des cellules avec les composés : 24h
Composés testés :
   - BLUMILlGHT^{™} (BUTYLENE GLYCOL and WATER and THEOBROMA CACAO (COCOA) SEED EXTRACT) à 0.05%
   - FILMEXEL^{®} (CAESALPINIA SPINOSA EXTRACT and KAPPAPHYCUS ALVAREZII EXTRACT and WATER) à 0.25%
   - BLUMILlGHT^{™} à 0.05% + FILMEXEL^{®} à 0.25%

### Résultats

Le tableau 1 ci-dessous retranscrit les variations d'activités pour les gènes étudiés.

Les conditions expérimentales retenues sont les suivantes :
- Chaque essai est réalisé en triplicate (3 non traités et 3 traités dans les mêmes conditions) ;
- Le test-F de Fischer est d'abord appliqué en comparant les deux matrices de données. Lorsque la valeur est supérieure à α = 0,05 alors la variance du test t de Student est de 2, lorsque le test-F de Fischer est inférieur à α = 0,05, alors la variance sera égale à 3.
- Les variations transcriptionnelles retenues (significatives) seront celles qui correspondent à un test t de Student inférieur ou égal à α (ou p) = 0,05. En gris clair les inhibitions et en gris foncé les stimulations (voir ci-dessous) le fond blanc indiquant les variations non significatives c'est-à-dire celles correspondant à un test t de Student supérieur à alpha (ou p) = 0,05.
- L'indication « ND » pour « non déterminé » signifie que le gène à une activité transcriptionnelle trop faible pour être quantifiée par la méthodologie TLDA utilisée.

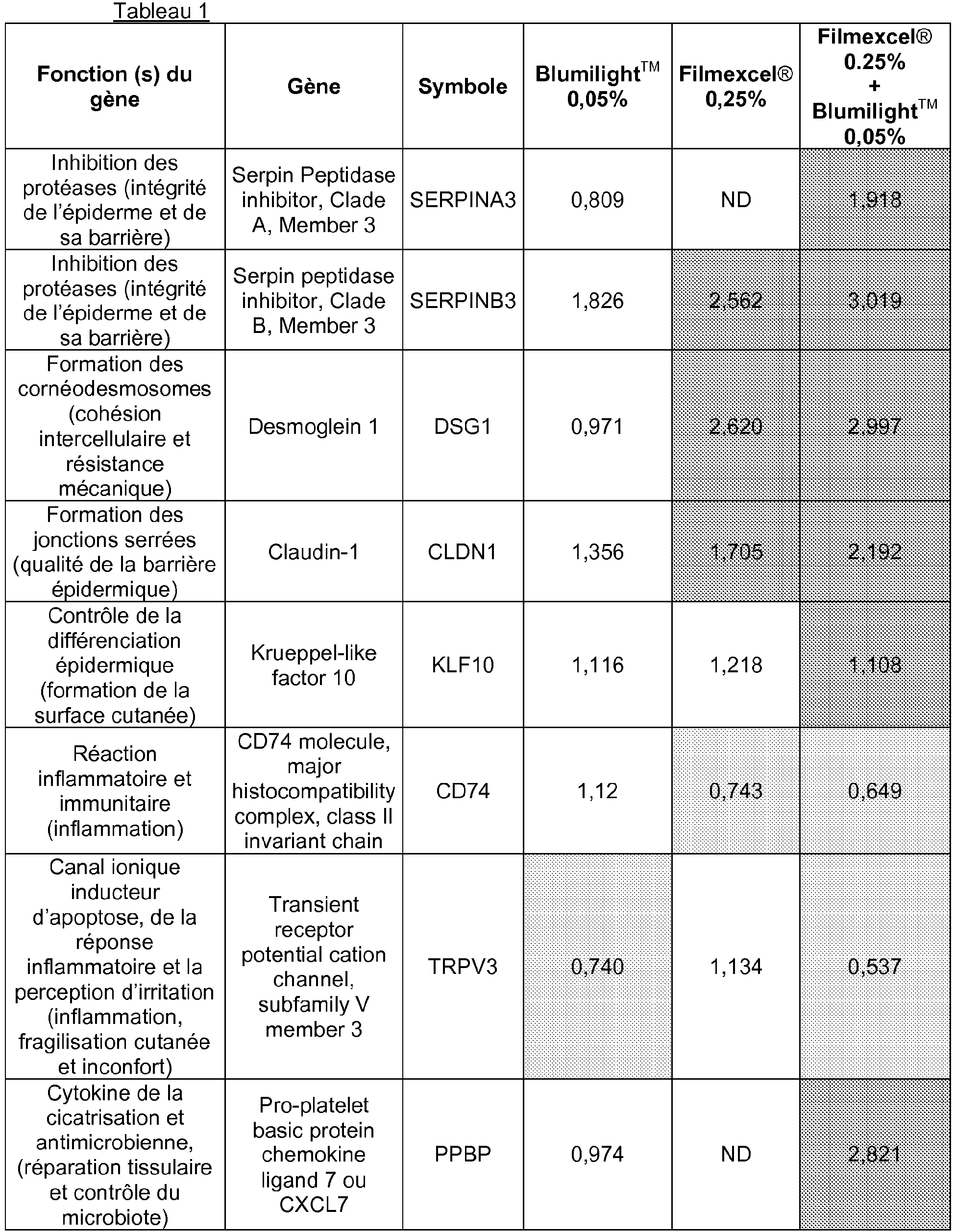

Ces données mettent en évidence, pour les associations testées, un effet synergique permettant globalement de renforcer et préserver les structures de l'épiderme en particulier :
- de favoriser l'intégrité des structures protéiques de la peau impliquées dans son maintien, dans l'effet astringent sur les pores et le comblement des reliefs de la peau, en empêchant la dégradation de ces structures protéiques par l'activation des gènes des inhibiteurs de protéases comme la SERPINA3, SERPINB3,
- de renforcer la résistance mécanique de l'épiderme en stimulant l'activité transcriptionnelle du gène DSG1 impliqué dans la formation des cornéodesmosomes, (structure protéique liant les cellules entre elles),
- d'améliorer la performance de la barrière cutanée en stimulant l'activité transcriptionnelle du gène CLDN1 impliqué dans la formation des jonctions serrées intercellulaires qui limitent le passage de l'eau intercellulaire (perte insensible en eau) et maintiennent les lipides épidermiques à la surface cutanée,
- de favoriser le bon développement de l'épiderme en particulier en stimulant l'activité transcriptionnelle du gène KLF10 impliqué dans la différenciation des kératinocytes épidermiques et en stimulant celle du gène PPBP (alias CXCL7) impliqué dans la cicatrisation et la vascularisation,
- de moduler la composante sensorielle de l'épiderme en particulier de pondérer la sensation d'inconfort due à l'inflammation par inhibition de l'activité transcriptionnelle du gène TRPV3, également impliqué dans la fragilisation de l'épiderme (apoptose des cellules),
- d'augmenter le contrôle exercé par la peau sur le microbiote en stimulant l'activité transcriptionnelle du gène CXCL7, cytokine antimicrobienne bactéricide et anti-fongique.

L'association selon l'invention d'un extrait de *Caesalpinia spinosa* et d'un extrait de *Kappaphycus alvarezii* (Filmexel^{®}) avec hydrolysat de fèves de *Theobroma cacao L* (Blumilight^{™}) permet donc de maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau, limiter les conséquences cutanées et sensorielles d'un environnement hypoxique ou inflammatoire, diminuer la perte de fermeté, diminuer le relâchement des pores et la formation d'imperfections du relief cutané (rides et ridules), favoriser et/ou améliorer l'homogénéité de répartition d'un produit de maquillage.

### Exemple 3 : Formulations cosmétiques

Les formulations cosmétiques sont préparées selon les méthodes classiques de formulations. Les pourcentages sont exprimés en pourcentages pondéraux d'ingrédient (matière première) par rapport au poids total de la composition.

### Une crème de jour Hydratante pour peau mixte à grasse:

| | |
|---|---|
| *GLYCEROL VEGETAL* | |
| (Glycerine) | 6.0% |
| *BLUMILlGHT^{™}* | |
| *(Butylène glycol and Water and Theobroma cacao (cocoa) seed extract)* | *0.10%* |
| FILMEXEL^{®} | |
| (*Caesalpinia spinosa fruit extract and Kappaphycus alvarezii extract and water*) | 0.50% |
| VITACTYL CLAIR 2 MB | |
| (Malva sylvestrys (Mallow) extract and phenoxyethanol and water | 3.0% |
| RENOHYAL | |
| (Sodium Hyaluronate) | 0.10% |
| Isononyl isononanoate | 10.0% |
| Steareth-2 | 12.5% |
| Glycéryl stearate | 1.1% |
| Alcool stéarylique | 5.0% |
| Butylène glycol | 3.0% |
| Glycérine | 2.0% |
| Conservateur | 0,5% |
| Eau, qsp | 100% |

### Un gel pour peau mixte :

| | |
|---|---|
| *GLYCEROL VEGETAL* | |
| *(Glycerine)* | 6.0% |
| *BLUMILlGHT^{™}* | |
| *(Butylène glycol and Water and Theobroma cacao (cocoa) seed extract)* | 0.50% |
| FILMEXEL^{®} | |

| | |
|---|---|
| (*Caesalpinia spinosa fruit extract and Kappaphycus alvarezii extract and water*) | 1.00% |
| RENOHYAL | |
| (Sodium Hyaluronate) | 0.1% |
| Glycol | 3.0% |
| Polymère d'AMPS | 3.0% |
| Huile minérale | 2.0% |
| Polyéthylène glycol | 1.5% |
| Conservateur | 0.5% |
| Concentré de parfum | 0,3% |
| Eau, Qsp | 100% |

### Un Fond de Teint SPF

| | |
|---|---|
| *BLUMILlGHT^{™}* | |
| *(Butylène glycol and Water and Theobroma cacao (cocoa) seed extract)* | *0.10%* |
| FILMEXEL^{®} | |
| (*Caesalpinia spinosa fruit extract and Kappaphycus alvarezii extract and water*) | 0.50% |
| UV-TITANE M160 | |
| *(Titanium dioxide, TiO₂ (nano))* | 2.0% |
| OXYDE ZINC | |
| *(Zinc oxide, Cl* 77947) | 3.0% |
| OXYDES DE FER ET DIOXYDE DE TITANE | |
| *(Black Cl* 77499, *red Cl* 77491, *yellow Cl* 77492, *White Cl* 77891) | 18.0% |
| Cyclopentasiloxane et cyclohexasiloxane | 5.0% |
| Cetyl diméthicone | 1.0% |
| Caprylic/capric triglycérides | 2.2 % |
| Octyl stéarate | 1.4 % |
| Huile minérale | 3.5 % |
| Cire d'abeille | 0.8 % |
| Polyméthacrylate de méthyle | 1.1% |
| Concentré de parfum | 0.1 % |
| Eau, qsp | 100% |

### Masque séborégulateur

| | |
|---|---|
| *GLYCEROL VEGETAL* | |
| *(Glycerine)* | 6.0% |
| *BLUMILlGHT^{™}* | |
| *(Butylène glycol and Water and Theobroma cacao (cocoa) seed extract)* | *1.00%* |
| FILMEXEL^{®} | |
| (*Caesalpinia spinosa fruit extract and Kappaphycus alvarezii extract and water*) | 0.50% |
| MINERALIS Gu / Zn | |
| (Zinc Gluconate) | 0.2% |
| 5 ALPHA AVOCUTA | |
| (Butyl avocadate, propyl gallate) | 1.0% |
| Glycerine | 5.0% |
| Cetearyl alcohol & cetearyl glucoside | 3.0% |
| Myristyl alcohol & myristyl glucoside | 2.0% |
| Octyldodecanol | 3.0% |
| Pentylene glycol | 1 .0% |
| Alcohol cetearylic | 0.5% |
| sodium Polyacrylate | 0.5% |
| Stearoyl glutamate of sodium | 0.3% |
| Acrylate/C 10-30 alkylacrylate crosspolymer | 0.2% |
| Gomme xanthane | 0.1% |
| disodic EDTA | 0.1% |
| Parfum | 0.1% |
| Eau qsp | 1 00% |

### Un Fond de Teint SPF

| | |
|---|---|
| *BLUMILlGHT^{™}* | |
| *(Butylène glycol and Water and Theobroma cacao (cocoa) seed extract)* | 0.10% |
| FILMEXEL^{®} | |
| (*Caesalpinia spinosa fruit extract and Kappaphycus alvarezii extract and water*) | 0.50% |
| *ECOSKIN^{®}* | |

| | |
|---|---|
| *(Alpha-glucan oligosaccharide and Polymnia sonchifolia root juice and Maltodextrin* | |
| *and Lactobacillus)* | 0.50% |
| UV-TITANE M160 | |
| *(Titanium dioxide, TiO₂ (nano))* | 2.0% |
| OXYDE ZINC | |
| *(Zinc oxide, Cl* 77947) | 3.0% |
| OXYDES DE FER ET DIOXYDE DE TITANE | |
| (*Black Cl* 77499, *red Cl* 77491, *yellow Cl* 77492, *White Cl* 77891) | 16.0% |
| Cyclopentasiloxane et cyclohexasiloxane | 5.0% |
| Cetyl diméthicone | 1.0% |
| Caprylic/capric triglycérides | 2.2 % |
| Octyl stéarate | 1.4 % |
| Huile minérale | 3.5 % |
| Cire d'abeille | 0.8 % |
| Polyméthacrylate de méthyle | 1.1% |
| Concentré de parfum | 0.1 % |
| Eau, qsp | 100% |

## Revendications

1. Composition cosmétique comprenant, dans un milieu physiologiquement acceptable, au moins:
a) un agent cosmétique constitué par des galactomannanes de masses molaires comprises entre 5 et 630 kDa, de préférence entre 5 et 120kDa, de préférence encore entre 8 et 80kDa, et des galactanes sulfatés réticulés de masses molaires comprises entre 7 et 3000kDa, de préférence entre 7 et 1100kDa, de préférence encore entre 8 et 200kDa, et
b) un hydrolysat de fèves de *Theobroma cacao L.*

2. Composition cosmétique selon la revendication 1, **caractérisée en ce que** l'agent cosmétique est constitué de galactomannanes obtenus par hydrolyse de galactomannanes natifs de Tara (*Caesalpinia spinosa*)*,* de guar (*Cyamopsis tetragonoloba*)*,* de caroube (*Ceratonia siliqua*), mais aussi de séné (Cassia *angustifolia*), de cassier (Cassia *fistula*)*,* de cassia (Cassia *obtusifolia ou Cassia tora*), de caroube chinois (*Gleditsia sinensis*)*,* de févier (*Gieditsia triacanthos*)*,* de sophora (*Sophora japonica*) et/ou de fenugrec (*Trigonella foenum-graecum*)*,* de préférence de *Caesalpinia spinosa* ; et de galactanes sulfatés réticulés obtenus par hydrolyse de galactanes sulfatés natifs de carraghénanes (de *Kappaphycus alvarezii,* de *Kappaphycus striatum, d'Eucheuma cottonii, d'Eucheuma spinosum,* de *Chondrus crispus,* de *Gigartina skottsbergii,* de *Sacrothalia crsipata*)*,* ou de *Fucellaria fastigiata,* d'Agar (*Gelidium sesquipedale*) ou des algues (*Polysiphonia lanosa* ou *Codium fragile*)*,* de préférence de *Kappaphycus alvarezii.*

3. Composition cosmétique selon la revendication 1 ou 2, **caractérisée en ce que** l'agent cosmétique a) est constitué de 60 à 90% de galactomannanes et 10 à 40% de galactanes sulfatés réticulés.

4. Composition cosmétique selon l'une des revendications 1 à 3, **caractérisée en ce que** l'agent cosmétique est constitué de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises notamment entre 1 et 150kDa.

5. Composition cosmétique selon l'une quelconque des revendications 1 à 4, **caractérisée en ce que** l'agent cosmétique a) est présent dans la composition en une teneur allant de 0,01% à 2% en poids de matière active par rapport au poids total de la composition, préférentiellement 0,1% à 0,1%, et préférentiellement encore de 0,4% à 0,8% en poids de matière active par rapport au poids total de la composition.

6. Composition cosmétique selon l'une quelconque des revendications 1 à 5, **caractérisée en ce que** l'hydrolysat de fèves de *Theobroma cacao L* est un hydrolysat peptidique et osidique de fèves de *Theobroma cacao L,* comprenant notamment peptides et osides présentant un poids moléculaire compris entre 200 Da et 10 kDa.

7. Composition cosmétique selon l'une quelconque des revendications 1 à 6, **caractérisée en ce que** l'hydrolysat de fèves de *Theobroma cacao L.* est présent dans la composition en une teneur allant de 0,0005 à 0,025% en poids de matière active par rapport au poids total de la composition, préférentiellement de 0,001 à 0,01% en poids de matière active par rapport au poids total de la composition.

8. Composition cosmétique selon l'une quelconque des revendications 1 à 7, **caractérisée en ce qu'**elle comprend en outre un prébiotique et un probiotique.

9. Composition cosmétique selon l'une quelconque des revendications 1 à 8, **caractérisée en ce qu'**elle se présente sous la forme d'un produit de soin et/ou d'un produit de maquillage pour la peau, les lèvres ou les yeux, en particulier pour le teint.

10. Procédé cosmétique de soin et/ou de maquillage des matières kératiniques, en particulier de la peau, des lèvres et/ou des yeux, comprenant au moins l'application sur lesdites matières kératiniques d'une composition cosmétique de soin et/ou de maquillage telle que définie dans l'une quelconque des revendications précédentes, en particulier d'une composition de maquillage pour le teint, les lèvres ou les yeux, de préférence pour le teint.

11. Procédé cosmétique de soin et/ou de maquillage selon la revendication 10, destiné à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, et/ou favoriser et/ou améliorer la tenue du maquillage et l'homogénéité de son dépôt.

12. Utilisation cosmétique non thérapeutique d'au moins un agent cosmétique constitué par des galactomannanes de masses molaires comprises entre 5 et 630kDa et des galactanes sulfatés réticulés de masses molaires comprises entre 7 et 3000kDa, et d'au moins un hydrolysat de fèves de *Theobroma cacao L.* tels que définis dans l'une quelconque des revendications 1 à 8, comme association destinée à maintenir l'écosystème cutané et/ou maintenir l'équilibre du microbiote cutané, en particulier la diversité microbienne, renforcer la barrière cutanée et son film hydrolipidique, améliorer l'intégrité et la résistance de la peau aux stress, en particulier limiter les conséquences cutanées d'un environnement hypoxique, favoriser la desquamation et/ou le renouvellement épidermique, diminuer la perte de fermeté, diminuer le relâchement des pores, diminuer les irrégularités de surface, améliorer l'homogénéité du teint, et/ou favoriser et/ou améliorer la tenue du maquillage et l'homogénéité de son dépôt.

13. Au moins a) un agent constitué par des galactomannanes de masses molaires comprises entre 5 et 630kDa et des galactanes sulfatés réticulés de masses molaires comprises entre 7 et 3000kDa, en particulier de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises notamment entre 1 et 150kDa, et b) au moins un hydrolysat de fèves de *Theobroma cacao Len* particulier un hydrolysat peptidique et osidique de fèves de *Theobroma cacao L,* comprenant notamment des peptides et osides présentant un poids moléculaire compris entre 200 Da et 10 kDa, ou une composition contenant a) et b), pour une utilisation pour traiter et/ou diminuer les désordres liés à l'hyper-séborrhée, en particulier l'inflammation sébum-dépendante et/ou la prolifération bactérienne.

## Patentansprüche

1. Kosmetische Zusammensetzung, die in einem physiologisch verträglichen Medium mindestens enthält:
a) ein kosmetisches Mittel bestehend aus Galactomannanen mit Molmassen zwischen 5 und 630 kDa, vorzugsweise zwischen 5 und 120 kDa, besonders bevorzugt zwischen 8 und 80 kDa, und vernetzten sulfatierten Galactanen mit Molmassen zwischen 7 und 3000 kDa, vorzugsweise zwischen 7 und 1100 kDa, besonders bevorzugt zwischen 8 und 200 kDa, und
b) ein Hydrolysat der Bohnen von *Theobroma cacao L*.

2. Kosmetische Zusammensetzung nach Anspruch 1, **dadurch gekennzeichnet, dass** das kosmetische Mittel aus Galactomannanen besteht, die durch Hydrolyse von nativen Galactomannanen von Tara *(Caesalpinia spinosa),* von Guar (*Cyamopsis tetragonoloba*), von Johannisbrot (*Ceratonia siliqua*)*,* aber auch von Senna (*Cassia angustifolia*)*,* Schwarzer Johannisbeere (*Cassia fistula*), Cassia (*Cassia obtusifolia* oder *Cassia tora*)*,* Chinesischer Gleditschie *(Gleditsia sinensis),* Feigenbaum (*Gieditsia triacanthos*)*,* Sophora *(Sophora japonica)* und/oder Bockshornklee (*Trigonella foenum-graecum*)*,* vorzugsweise *Caesalpinia spinosa* gewonnen werden; und aus vernetzten sulfatierten Galactanen besteht, die durch Hydrolyse von nativen sulfatierten Galaktanen von Carrageenanen (aus *Kappaphycus alvarezii, Kappaphycus striatum, Eucheuma cottonii, Eucheuma spinosum, Chondrus crispus, Gigartina skottsbergii, Sacrothalia crsipata*)*,* oder von *Fucellaria fastigiata,* von Agar (*Gelidium sesquipedale*) oder von Algen *(Polysiphonia lanosa* oder *Codium fragile),* vorzugsweise von *Kappaphycus alvarezii,* gewonnen werden.

3. Kosmetische Zusammensetzung nach Anspruch 1 oder 2, **dadurch gekennzeichnet, dass** das kosmetische Mittel a) aus 60 bis 90% Galactomannanen und 10 bis 40% vernetzten sulfatierten Galactanen besteht.

4. Kosmetische Zusammensetzung nach einem der Ansprüche 1 bis 3, **dadurch gekennzeichnet, dass** das kosmetische Mittel aus Galactomannanen, die aus *Caesalpinia spinosa* gewonnen werden, mit Molmassen zwischen 1 und 150 kDa, und aus vernetzten sulfatierten Galactanen besteht, die aus *Kappaphycus alvarezii* gewonnen werden, mit Molmassen insbesondere zwischen 1 und 150 kDa.

5. Kosmetische Zusammensetzung nach einem der Ansprüche 1 bis 4, **dadurch gekennzeichnet, dass** das kosmetische Mittel a) in der Zusammensetzung in einem Gehalt von 0,01 bis 2 Gew.-% des Wirkstoffs, bezogen auf das Gesamtgewicht der Zusammensetzung, vorzugsweise von 0,1 bis 0,1 Gew.-% und besonders bevorzugt von 0,4 bis 0,8 Gew.-% des Wirkstoffs, bezogen auf das Gesamtgewicht der Zusammensetzung, vorliegt.

6. Kosmetische Zusammensetzung nach einem der Ansprüche 1 bis 5, **dadurch gekennzeichnet, dass** das Hydrolysat der Bohnen von *Theobroma cacao L ein* Peptid- und Osidhydrolysat der Bohnen von *Theobroma cacao L ist,* das insbesondere Peptide und Oside mit einem Molekulargewicht zwischen 200 Da und 10 kDa enthält.

7. Kosmetische Zusammensetzung nach einem der Ansprüche 1 bis 6, **dadurch gekennzeichnet, dass** das Hydrolysat der Bohnen von *Theobroma cacao L* in der Zusammensetzung in einem Gehalt von 0,0005 bis 0,025 Gew.-% des Wirkstoffs, bezogen auf das Gesamtgewicht der Zusammensetzung, vorzugsweise von 0,001 bis 0,01 Gew.-% des Wirkstoffs, bezogen auf das Gesamtgewicht der Zusammensetzung, vorliegt.

8. Zusammensetzung nach einem der Ansprüche 1 bis 7, **dadurch gekennzeichnet, dass** sie ferner ein Präbiotikum und ein Probiotikum enthält.

9. Kosmetische Zusammensetzung nach einem der Ansprüche 1 bis 8, **dadurch gekennzeichnet, dass** sie in Form eines Pflege- und/oder Schminkprodukts für die Haut, die Lippen oder die Augen, insbesondere für den Teint, vorliegt.

10. Kosmetisches Verfahren zum Pflegen und/oder Schminken von Keratinmaterialien, insbesondere der Haut, der Lippen und/oder der Augen, umfassend mindestens das Auftragen einer kosmetischen Zusammensetzung zur Pflege und/oder zum Schminken nach einem der vorstehenden Ansprüche auf die Keratinmaterialien, insbesondere eine Zusammensetzung zum Schminken für den Teint, die Lippen oder die Augen, vorzugsweise für den Teint.

11. Kosmetisches Pflege- und/oder Schminkverfahren nach Anspruch 10 zur Erhaltung des Ökosystems der Haut und/oder zur Aufrechterhaltung des Gleichgewichts der Hautmikrobiota, insbesondere der mikrobiellen Vielfalt, zur Stärkung der Hautbarriere und ihres Hydrolipidfilms, zur Verbesserung der Integrität und Widerstandsfähigkeit der Haut gegen Stress, insbesondere zur Begrenzung der kutanen Folgen einer hypoxischen Umgebung, zur Förderung der Abschuppung und/oder zur Erneuerung der Epidermis, zur Verringerung des Verlustes an Festigkeit, zur Verringerung der Erschlaffung der Poren, zur Verringerung von Oberflächenunregelmäßigkeiten, zur Verbesserung der Gleichmäßigkeit des Teints und/oder zur Förderung und/oder Verbesserung der Haltbarkeit des Make-ups und der Gleichmäßigkeit seines Auftrags.

12. Nicht-therapeutische kosmetische Verwendung mindestens eines kosmetischen Mittels, das aus Galactomannanen mit Molmassen zwischen 5 und 630 kDa und vernetzten sulfatierten Galactanen mit Molmassen zwischen 7 und 3000 kDa und mindestens einem Hydrolysat der Bohnen von *Theobroma cacao L.* nach einem der Ansprüche 1 bis 8 besteht, als Kombination zur Erhaltung des Ökosystems der Haut und/oder zur Aufrechterhaltung des Gleichgewichts der Hautmikrobiota, insbesondere der mikrobiellen Vielfalt, zur Stärkung der Hautbarriere und ihres Hydrolipidfilms, zur Verbesserung der Integrität und Widerstandsfähigkeit der Haut gegen Stress, insbesondere zur Begrenzung der kutanen Folgen einer hypoxischen Umgebung, zur Förderung der Abschuppung und/oder zur Erneuerung der Epidermis, zur Verringerung des Verlustes an Festigkeit, zur Verringerung der Erschlaffung der Poren, zur Verringerung von Oberflächenunregelmäßigkeiten, zur Verbesserung der Gleichmäßigkeit des Teints und/oder zur Förderung und/oder Verbesserung der Haltbarkeit des Make-ups und der Gleichmäßigkeit seines Auftrags.

13. Mindestens a) ein Mittel, das aus Galactomannanen mit Molmassen zwischen 5 und 630 kDa und vernetzten sulfatierten Galactanen mit Molmassen zwischen 7 und 3000 kDa, insbesondere Galactomannanen, die aus *Caesalpinia spinosa* gewonnen werden, mit Molmassen zwischen 1 und 150 kDa, und vernetzten sulfatierten Galactanen, die aus *Kappaphycus alvarezii* gewonnen werden, mit Molmassen von insbesondere zwischen 1 und 150 kDa, besteht, und b) mindestens ein Hydrolysat der Bohnen von *Theobroma cacao L,* insbesondere ein Peptid- und Osidhydrolysat der Bohnen von *Theobroma cacao L,* das insbesondere Peptide und Oside mit einem Molekulargewicht zwischen 200 Da und 10 kDa enthält, oder eine Zusammensetzung, die a) und b) enthält, zur Verwendung bei der Behandlung und/oder Verringerung von Erkrankungen, die mit Hyperseborrhoe verbunden sind, insbesondere talgabhängiger Entzündung und/oder bakterieller Proliferation.

## Claims

1. A cosmetic composition comprising, in a physiologically acceptable medium, at least:
a) a cosmetic agent consisting of galactomannans with molar masses comprised between 5 and 630 kDa, preferably between 5 and 120 kDa, and even more preferably between 8 and 80 kDa, and crosslinked sulfated galactans with molar masses comprised between 7 and 3000 kDa, preferably between 7 and 1100 kDa, and even more preferably between 8 and 200 kDa, and
b) a hydrolysate of *Theobroma cacao* L. beans.

2. The cosmetic composition as claimed in claim 1, **characterized in that** the cosmetic agent consists of galactomannans obtained by hydrolysis of native galactomannans of Tara *(Caesalpinia spinosa),* guar (*Cyamopsis tetragonoloba*), carob (*Ceratonia siliqua*), but also senna (*Cassia angustifolia*), cassia (*Cassia fistula*), cassia (*Cassia obtusifolia* or *Cassia tora*), Chinese locust bean (G*leditsia sinensis*)*,* honey locust (*Gieditsia triacanthos*)*,* sophora (*Sophora japonica*) and/or fenugreek (*Trigonella foenum-graecum*)*,* preferably *Caesalpinia spinosa;* and crosslinked sulfated galactans obtained by hydrolysis of native sulfated galactans of carrageenans (from *Kappaphycus alvarezii, Kappaphycus striatum, Eucheuma cottonii, Eucheuma spinosum, Chondrus crispus, Gigartina skottsbergii, Sacrothalia crispata*)*,* or *Fucellaria fastigiata,* Agar (*Gelidium sesquipedale*) or algae (*Polysiphonia lanosa* or *Codium fragile*)*,* preferably *Kappaphycus alvarezii.*

3. The cosmetic composition as claimed in claim 1 or 2, **characterized in that** the cosmetic agent a) consists of 60 to 90% galactomannans and 10 to 40% crosslinked sulfated galactans.

4. The cosmetic composition as claimed in one of claims 1 to 3, **characterized in that** the cosmetic agent consists of galactomannans obtained from *Caesalpinia spinosa* with molar masses comprised between 1 and 150 kDa, and of crosslinked sulfated galactans obtained from *Kappaphycus alvarezii* with molar masses comprised between 1 and 150 kDa in particular.

5. The cosmetic composition as claimed in any one of claims 1 to 4, **characterized in that** the cosmetic agent a) is present in the composition in a content ranging from 0.01% to 2% by weight of active material based on the total weight of the composition, preferentially 0.1% to 0.1%, and more preferentially from 0.4% to 0.8% by weight of active material based on the total weight of the composition.

6. The cosmetic composition as claimed in any one of claims 1 to 5, **characterized in that** the hydrolysate of *Theobroma cacao* L. beans is a peptide and monosaccharide hydrolysate of *Theobroma cacao* L. beans, comprising in particular peptides and monosaccharides having a molecular weight comprised between 200 Da and 10 kDa.

7. The cosmetic composition as claimed in any one of claims 1 to 6, **characterized in that** the hydrolysate of *Theobroma cacao* L. beans is present in the composition in a content ranging from 0.0005 to 0.025% by weight of active material based on the total weight of the composition, preferentially from 0.001 to 0.01% by weight of active material based on the total weight of the composition.

8. The cosmetic composition as claimed in any one of claims 1 to 7, **characterized in that** it further comprises a prebiotic and a probiotic.

9. The cosmetic composition as claimed in any one of claims 1 to 8, **characterized in that** it is in the form of a care product and/or a make-up product for the skin, lips or eyes, in particular for the complexion.

10. A cosmetic process for caring for and/or making-up keratinous materials, in particular the skin, lips and/or eyes, comprising at least the application to said keratinous materials of a cosmetic care and/or make-up composition as defined in any one of the preceding claims, in particular a make-up composition for the complexion, the lips or the eyes, preferably for the complexion.

11. The cosmetic care and/or make-up process as claimed in claim 10, for maintaining the cutaneous ecosystem and/or maintaining the balance of the cutaneous microbiota, in particular the microbial diversity, strengthening the skin barrier and its hydrolipidic film, improving the integrity and the resistance of the skin to stress, in particular limiting the cutaneous consequences of a hypoxic environment, promoting desquamation and/or epidermal renewal, reducing the loss of firmness, reducing the slackening of the pores, reducing surface irregularities, improving the evenness of the complexion, and/or promoting and/or improving the hold of make-up and the evenness of its application.

12. Non-therapeutic cosmetic use of at least one cosmetic agent consisting of galactomannans with molar masses comprised between 5 and 630 kDa and crosslinked sulfated galactans with molar masses comprised between 7 and 3000 kDa, and of at least one hydrolysate of *Theobroma cacao* L. beans as defined in any one of claims 1 to 8, as a combination for maintaining the cutaneous ecosystem and/or maintaining the balance of the cutaneous microbiota, in particular the microbial diversity, strengthening the skin barrier and its hydrolipidic film, improving the integrity and the resistance of the skin to stress, in particular limiting the cutaneous consequences of a hypoxic environment, promoting desquamation and/or epidermal renewal, reducing the loss of firmness, reducing the slackening of the pores, reducing surface irregularities, improving the evenness of the complexion, and/or promoting and/or improving the hold of make-up and the evenness of its application.

13. At least a) an agent consisting of galactomannans with molar masses comprised between 5 and 630 kDa and crosslinked sulfated galactans with molar masses comprised between 7 and 3000 kDa, in particular galactomannans obtained from *Caesalpinia spinosa* with molar masses comprised between 1 and 150 kDa, and crosslinked sulfated galactans obtained from Kappaphycus alvarezii with molar masses of in particular between 1 and 150 kDa, and b) at least one hydrolysate of *Theobroma cacao* L. beans, in particular a peptide and monosaccharide hydrolysate of *Theobroma cacao* L. beans, comprising in particular peptides and monosaccharides having a molecular weight comprised between 200 Da and 10 kDa, or a composition containing a) and b), for use in treating and/or reducing disorders associated with hyper-seborrhea, in particular sebum-dependent inflammation and/or bacterial proliferation.
